(19) Europäisches Patentamt
European Patent Office
Office européen des brevets

(11) Veröffentlichungsnummer: **0 450 117 A1**

# (12) EUROPÄISCHE PATENTANMELDUNG

(21) Anmeldenummer: **90106312.3**

(22) Anmeldetag: **02.04.90**

(51) Int. Cl.[5]: **A01N 47/44**, A61K 33/14, //(A01N47/44,59:08,31:02)

Ein Antrag gemäss Regel 88 EPÜ auf Berichtigung der Beschreibung liegt vor. Über diesen Antrag wird im Laufe des Verfahrens vor der Prüfungsabteilung eine Entscheidung getroffen werden (Richtlinien für die Prüfung im EPA, A-V, 2.2).

(43) Veröffentlichungstag der Anmeldung: **09.10.91 Patentblatt 91/41**

(84) Benannte Vertragsstaaten: **AT BE CH DE DK ES FR GB GR IT LI LU NL SE**

(71) Anmelder: **INFECTLESS S.A.**
**Palazzo Commerciale**
**CH-6535 Roveredo GR(CH)**

(72) Erfinder: **Good, Hans Dr.**
**Casella postale No. 63**
**CH-6535 Roveredo(CH)**

(74) Vertreter: **Pätzold, Herbert, Dr.**
**Steubstrasse 10**
**W-8032 Gräfelfing-München(DE)**

(54) **Ringerlösung und dessen Anwendung als bakterizid wirkendes lokales Wundbehandlungsmedikament.**

(57) Eine lactatfreie reine Ringerlösung, die zusätzlich 0,1 % eines Konzentrates gelöst enthält, das aus einer 20%-igen wäßrigen Polyhexamethylenbiguanidin-Hydrochlorid-Lösung besteht, in der pro 100 ml 1 g Polyethylenglykol mit einem Molekulargewicht von etwa 4000 gelöst ist, dient zur lokalen bakteriellen Dekontamination von Wunden auf einen keimfreien oder saprophytischen Zustand und wird damit als ein bakterizid wirkendes Wundbehandlungsmedikament verwendet.

EP 0 450 117 A1

Die Erfindung betrifft eine lactatfreie reine Ringerlösung mit Zusätzen, die sie zur Dekontamination von Wunden auf einen keimfreien oder saprophytischen Zustand und zur Aufrechterhaltung eines solchen Zustandes während der gesamten Heilungsdauer geeignet machen.

Insbesondere soll es sich um eine Ringerlösung mit solchen Zusätzen handeln, daß sie als bakterizid wirkendes Medikament zur Anwendung bei therapeutischen Verfahren für die lokale Behandlung von infizierten Wunden, für die lokale prophylaktische Behandlung von infektionsgefährdeten Wunden vor allem während der Operation und für die Sicherung der lokalen bakteriellen Dekontamination von Wunden während des Heilungsprozesses gleich gut geeignet ist.

Es sind die verschiedensten Wundbehandlungsmittel bekannt, die jedoch zur medikamentösen Applikation wegen ihrer mangelhaften Gewebeverträglichkeit, ihrer hohen Eiweißfehler (Beeinträchtigung der Bakterizidie in Gegenwart von Eiweiß, z.B. Wundsekret und Blut, ihrer teilweise ungenügenden Bakterizidie und wegen ihrer nachteiligen Eigenschaften, in mehr oder weniger großen Mengen vom Körper resorbiert zu werden, nicht geeignet sind bzw. nicht befriedigen. Es kommt noch hinzu, daß die Gewebeverträglichkeit solcher Mittel wegen zu hoher Oberflächenspannung mitunter nicht ausreicht.

Bei der Applikation von Jodophoren zur Wundbehandlung muß man mit der Resorption von Jod und damit mit der Gefahr von jodbedingten Störungen rechnen. Weiterhin weisen Jodophoren einen beträchtlichen Eiweißfehler auf. Eine Jodophor-Verdünnung von 1:10 zeigt im Labortest eine noch gute Bakterizidie, die aber in Gegenwart von 20 % Vollblut so gut wie vollständig oder weitgehend aufgehoben wird. Wegen der Gefahr von postoperativem Schockzustand sogar mit tödlichem Ausgang ist die intraabdominale Anwendung von Jodophoren gänzlich verlassen worden.

Chlorhexidin wirkt im wesentlichen nur bakteriostatisch und wird daher für lokale Wundbehandlung nicht mehr empfohlen.

Vielfach wird beklagt, daß sich Operationswunden vor pathogenen Keimen nicht ausreichend sicher schützen lassen, die von außen in die Wunde gelangen oder die als gutartige Keime im Körper vorhanden sind, aber durch die operativen pathogen werden können.

Das Infektionsrisiko bei Operationen ist daher nach wie vor hoch, was auch mit der zunehmenden Resistenz von Antibiotika zusammenhängt. Hinzu kommt, daß bisher gebräuchliche schwermetall-haltige, jodhaltige und aldehydhaltige Wundbehandlungsmittel wegen der Gefahr ernsthafter Nebenwirkungen nicht mehr zugelassen sind bzw. im zunehmenden Maße gemieden werden.

Aufgabe der Erfindung ist es daher, eine wäßrige Lösung anzugeben, die bei weitgehend geringem Eiweißfehler eine besonders hohe Bakterizidie aufweist, aber vom KÖrper auch im Kontakt mit offenen Wunden selbst bei längerer Anwendung von mehreren Wochen praktisch nicht resorbiert wird. Die Lösung soll daher vor allem als bakterizid wirkendes Medikament für lokale Wundbehandlungen verwendbar sein und die Verwendung von zusätzlichen systemischen Antibiotika senken oder vollständig erübrigen. Hierzu ist eine gute Gewebebenetzbarkeit und eine mit Ringerlösung vergleichbare hohe Gewebeverträglichkeit der erfindungsgemäßen Lösung erforderlich. Die Lösung soll außerdem schwermetall-, jod-, PVP- und aldehydfrei sein und sie soll die allgemeinen Auswirkungen einer Entzündung (Fieber, toxische Erscheinungen) ohne zusätzliche Applikationen von Antibiotika rasch mindern.

Die vorstehende Aufgabe wird erfindungsgemäß dadurch gelöst, daß eine lactatfreie, reine Ringerlösung verwendet wird, die zusätzlich 0,1 % eines Konzentrates gelöst enthält, das aus einer 20%-igen wäßrigen Polyhexamethylenbiguanidin-Hydrochlorid-Lösung besteht, in der pro 100 ml l g Polyethylenglykol mit einem Molekulargewicht von etwa 4.000 gelöst ist.

Ein Verfahren zur Herstellung von Polyhexamethylenbiguanidin-Hydrochlorid ist durch die deutsche Patentschrift 16 20 938 bekannt. Es besitzt die allgemeine Formel:

$$\left[ (CH_2)_6 - NH - \underset{\underset{NH}{\parallel}}{C} - NH - \underset{\underset{NH}{\parallel}}{C} - NH \right]_n \; n\ HCl$$

und ist unter dem Warennamen "Vantocil IB" der Firma Imperial Chemical Industries Ltd. für ein Flächeninfektionsmittel im Bereich der Lebensmittel-, Brauerei- und Getränkeindustrie auf dem Markt.

Das hier betreffende Polyethylenglykol ist unter dem Warennamen "LUTROL E 4000" der Fa. BASF auf dem Markt erhältlich.

Es war überraschend, daß die erfindungsgemäße Lösung eine hohe Bakterizidie aufweist, die gegen

alle medizinisch wichtigen Bakterien und Pilze eine hohe Wirksamkeit besitzt. Dabei weist die erfindungsgemäße Lösung in Gegenwart von 2 % Albumin keinen Eiweißfehler und in Gegenwart von 20%-igem Vollblut nur einen sehr geringen Eiweißfehler auf.

Wichtig ist dabei, daß die erfindungsgemäße Lösung kein Desinfektionsmittel herkömmlicher Art ist, das z.B. zur Händedesinfektion innerhalb kurzer Zeiten von 30 s bis maximal 1 min seine volle Wirksamkeit erreicht haben muß. Die erfindungsgemäße Lösung ist als Hautdesinfektionsmittel, insbesondere für die Vorbereitung von operativen Eingriffen ungeeignet.

Es ist das besondere Verdienst des Erfinders der vorliegenden Lösung, erstmals erkannt zu haben, daß die niedrigen Konzentrationen des ausgewählten Biguanids von 0,1 % in reiner, lactatfreier Ringerlösung zu einem resorptionsfreien Wundbehandlungsmedikament mit hoher Bakterizidie und ausgezeichneter Gewebeverträglichkeit führt, das selbst in Gegenwart von 20%-igem Vollblut einen praktisch vernachlässigbaren Eiweißfehler besitzt und in Gegenwart sehr geringer Mengen eines bestimmten Polyethylenglykols eine ausreichend hohe Gewebebenetzbarkeit erhält, wobei bei ausreichend langer lokaler Einwirkungszeit auf eine infizierte Wunde ein saprophytischer Keimzustand erhalten wird und sogar ein keimfreier Zustand erhalten werden kann.

Die Einwirkungszeit der erfindungsgemäßen Lösung auf die pathogenen Keime in infizierten Wunden beträgt mindestens 20 bis 60 min. Selbst bei wochenlanger Wundbehandlung mit der erfindungsgemäßen Lösung zeigt sich keine abnehmende Tendenz in der hohen Verträglichkeit. Auch konnte dabei keine nennenswerte Resorption der erfindungsgemäßen Lösung festgestellt werden. Erst wenn die erfindungsgemäße Konzentrationsmenge von 0,2 % überschritten wird, ist mit Wundheilungsstörungen zu rechnen. So zeigten Versuche mit einer 0,4 %-igen Lösung, daß sie wegen Inkompatibilität als bakterizid wirkendes Medikament nicht mehr geeignet ist.

Für die normale Anwendung ist die erfindungsgemäße Konzentration von 0,1 % völlig ausreichend. Nur in Ausnahmefällen, vor allem bei spez. gram-negativen Keimen wie sie vor allem in der Mundhöhle auftreten, wird eine erhöhte Konzentration des erfindungsgemäß verwendeten Biguanids von 0,2 % empfohlen, die jedoch nicht überschritten werden darf. Konzentrationen wesentlich unterhalb von 0,1 % weisen auch bei längerer Einwirkungszeit auf infizierte Wunden keine ausreichende Bakterizidie auf.

Entscheidend ist für die gute Wirksamkeit der erfindungsgemäßen Lösung, daß mit ihr eine Dekontamination der infizierten Wunde bis auf ein keimfreies oder saprophytisches Niveau weitgehend sichergestellt werden kann, daß dadurch eine ungestörte Heilung einsetzen kann und daß bei weiterer Anwendung der erfindungsgemäßen Lösung auf der Wunde der erzielte Dekontaminationsgrad auch aufrechterhalten werden kann, so daß die Voraussetzungen für eine optimale Wundheilung erzielt sind.

Hierdurch wird der Medikamentencharakter der erfindungsgemäßen Lösung im Gegensatz zu herkömmlichen Desinfektionsmitteln besonders deutlich, indem die erfindungsgemäße Lösung als Langzeitbehandlungsmittel anfangs für eine rasche und ausreichende Dekontamination der infizierten Wunden bis auf einem saprophytischen Keimzustand Sorge trägt und dieses während der gesamten Heilungsdauer bei ständiger oder intervallmäßiger Anwendung auch aufrechterhält. Entsprechend wirkt die erfindungsgemäße Lösung als prophylaktisches Mittel zum Schutz von Wunden vor zusätzlicher Verkeimung während des gesamten Heilungsprozesses. Hier kann es, wie gesagt, in Ausnahmefällen vorteilhaft sein, die etwas konzentriertere 0,2 %-ige Lösung zu verwenden.

Selbst bei längerer Anwendung der erfindungsgemäßen Lösung treten weder auf Wunden noch auf der Haut lokale Reizerscheinungen auf. Die Lösung besitzt eine lokale wie auch eine allgemeine symptomlose Verträglichkeit.

Die erfindungsgemäße Lösung hat sich für folgende Anwendngen besonders bewährt.

Wundspülungen von akuten und chronischen Knochen- und Weichteilinfektionen;
Spülungen von Herden als Vorbereitung zur Herdausräumung;
Spülungen von debridierten Herden, um auf einem optimal dekontaminierten Milieu die definitiven chirurgischen Heilmaßnahmen durchführen zu können, wie besonders das Einsetzen freier Knochentransplantate (Spongiosa), aber auch gestielte Weichteil- und Knochentransplantate;
Wundspülungen während der Operation, insbesondere auch zur Bekämpfung körpereigener Keime, die nach dem Freiwerden im Operationsgebiet pathogen werden können (Hospitalismus);
zur Abdeckung von postoperativ offengelassenen Wunden, z.B. mit Longetten, die mit der erfindungsgemäßen Lösung getränkt sind;
Spülungen und der Einsatz von mit erfindungsgemäßer Lösung getränkter Tamponaden in der zahnärztlichen Chirurgie;
peroperative Spülungen bei infektionsgefährdeten Eingriffen wie Osteosynthesen, beim Einsetzen und Wechsel von Endoprothesen, bei Wundversorgungen, bei Bauchdeckenwunden nach Verschluß des Peritoneums.

Bei völlig symptomloser Verträglichkeit hat sich die erfindungsgemäße 0,1 %-ige Lösung auch bei intraabdominalen Spülungen als wirksam erwiesen und zwar präventiv nach Magen- und Darmresektionen und therapeutisch nach Cholezystektomie bei Cholecysisitis actua. In keinem Fall waren peritonische Komplikationen (Restabszesse) zu beobachten. Die Spülmenge soll hierbei 1000 ml nicht überschreiten und die Spüldauer soll maximal 15 bis 20 min betragen. Anschließend soll mit Ringerlösung nachgespült werden. Bei einer intraabdominalen Spülung mit begrenzter Spülmenge wurde ebenfalls keine Resorption nachgewiesen. Eine derartige Anwendung kann in Ausnahmefällen auch mit einer 0,2 %-igen erfindungsgemäßen Lösung erfolgen. Die intraabdominale Anwendung der erfindungsgemäßen Lösung besitzt daher wesentliche Vorteile auch gegenüber Jodophoren, die wegen Jod-bedingter Störungen für diesen Zweck immer mehr gemieden werden.

Bei offenen Spül-Saugdrainagen wird die Tropf-Instillation mit Ringerlösung 1 bis 3 Mal unterbrochen und eine Tropf-Instillation mit erfindungsgemäßer Lösung dazwischengeschaltet.

Zahlreiche Versuche haben gezeigt, daß auch schwerst infizierte Wunden allein nur mit lokaler Applikation der erfindungsgemäßen Lösung erfolgreich behandelt werden konnten. Dabei konnte gezeigt werden, daß der Verbrauch von Antibiotika in der septischen Chirurgie beträchtlich herabgesetzt werden konnte. Die intermittierende Anwendung von erfindungsgemäßer Lösung mit reiner, lactatfreier Ringerlösung hat sich in vielen Fällen besonders gut bewährt. Die ausgezeichnete Gewebe- und Allgemeinverträglichkeit hat in einr großen Zahl von Anwendungsfällen zu ausgezeichneten Ergebnissen geführt, wenn infizierte Wunden außerhalb des Abdomens 2 bis 3 Mal täglich 20 bis 60 min lang, z.B. mit einer Gaze bedeckt wurden, die mit erfindungsgemäßer Lösung getränkt war. Jeweils anschließend erfolgte eine Nachspülung und Weiterbehandlung mit reiner lactatfreier Ringerlösung. Entsprechend waren wochenlange Anwendungen keine Seltenheit.

Es konnte weiterhin gezeigt werden, daß in einer Reihe von Osteitis-Fällen jeweils die lokalen Entzündungserscheinungen auffallend rasch zurückgingen, wenn bereits vorhandene Zugänge zu den Herden (Fisteln) mit der erfindungsgemäßen 0,1 %-igen Lösung gespült wurden. Dabei verschwanden insbesondere auch bei Pseudomonas aeruginosa die üblen Geruchsbildungen.

Weiterhin konnte beobachtet werden, daß bei Anwendung der erfindungsgemäßen Lösung die allgemeinen Auswirkungen einer Entzündung (Fieber, toxische Erscheinungen) rasch verschwinden, auch wenn kein systemisches Antibiotika verabreicht worden ist.

Besonders beeindruckend war die dekontaminierende Wirkung der erfindungsgemäßen Lösung bei Herden, die unausgeräumt mitsamt nekrotischem Inhalts mit der Lösung bespült wurden und dabei sogar keimfrei geworden sind, was vergleichsweise mit einer Bespülung mit Antibiotika nicht beobachtet werden konnte.

Wegen ihrer Avaskularität bildet die Spongiosa ein besonders günstiges Kriterium, um zu zeigen, welchen vorteilhaften Einfluß die erfindungsgemäße Lösung auf den anspruchsvollen Vorgang der Spongiosa-Einheilung hat. So konnten in einer Reihe von Spongiosa-Fällen ohne Ausnahmen bei Anwendung der erfindungsgemäßen Lösung ohne geringste Störung Spongiosa-Einlagen quantitativ vollständig eingeheilt werden. Die durchwegs sichere Einheilung der Spongiosa sowohl im Wirtbett als auch im Rahmen einer offenen Wundbehandlung kann als wesentliches Indiz dafür gewertet werden, daß durch die Bedeckung der Wunden mit Gazen, die mit einer erfindungsgemäßen Lösung getränkt sind, ein für die Wundheilung optimaler

Dekontaminationsgrad nicht nur erreicht, sondern auch über lange Zeit hin aufrechterhalten werden kann. Dadurch werden aber erst die wichtigen Voraussetzungen für eine störungsfreie Einheilung der Spongiosa-Einlagen erfüllt, d.h. die sichere Verhinderung einer Infektion und der damit verbundenen Störung der Revaskularisation des Spongiosagefüges. So sind auch in keinem einzigen Fall Abstossungen von Spongiosanteilen beobachtet worden, zudem hat auch die ununterbrochene Benetzung der Wunden mit der erfindungsgemäßen Lösung die Heilvorgänge in keiner Weise beeinträchtigt. Nicht zuletzt hat die erfindungsgemäße Lösung als solche auch die Erfordernisse eines adäquanten Feuchthaltens der Wunde erfüllt.

Auch bei debridierter ausreichend stabiler Kortikalis, die als solcher offengelassen wurde, konnte durch Bedeckung mit lösungsgetränkter Gaze ein Dekontaminationsgrad aufrechterhalten werden, der in allen Fällen zu einem spontanen Wundverschluß führte, wenn die Schrumpfungsmöglichkeit der Weichteile genügend war.

Die bakteriologischen Abstriche der postoperativ offen belassenen Wunden zeigten eine gute Übereinstimmung mit dem klinisch vermuteten Dekontaminationsgrad. In 3/4 aller Spongiosa-Fällen waren bereits die ersten postoperativen Wundabstriche oder der Inhalt von Saugdrainagen keimfrei; bei den "Kortikalis"-Fällen waren es 4/5.

Bei den übrigen Fällen blieben die Abstiche zunächst noch positiv, um erst später negativ zu werden. Dies war meistens bei Wunden der Fall, bei denen die Selbstreinigung der Granulationen verzögert verlief.

Wichtig ist der Hinweis, daß bei diesen Wunden die Einheilung der offenliegenden Spongiosa oder Kortikalis genauso störungsfrei verlief wie bei sauberen Granulationen.

An einem Kollektiv von 50 Fällen sind unter Behandlung mit erfindungsgemäßer Lösung (Spülung der Herde in der Vorbereitungsphase, peroperativ, Abdeckung der postoperativen Wunden mit lösungsgetränkten Longuetten) die folgenden Erreger nach unterschiedlichen Einwirkungszeiten zum Verschwinden gebracht worden:

Staph. aureus
verschiedene Streptokokken (Enterokokken)
Clostridium sphenoides
Pseudomonas aeruginosa
Serratus marcescens
Staph. epidermidis
E. Coli
Proteus morganii u. vulgaris
Klebsiella
Aeromonas hydrophilia
anaerobe Corynebakterien
Achromobacter agglomerans

Weitere Erkenntnisse ergaben sich bei der Behandlung von akuten Weichteilphlegmonen, bei denen eine Abszessbildung noch wenig oder nicht abgegrenzt war. Die inzidierten Herde wurden jeweils mittels stumpfer Kanüle mit erfindungsgemäßer Lösung kurz gespült. Dekontamination und damit verbundener Rückgang der lokalen und allgemeinen Entzündungserscheinungen erfolgte auffallend rasch. Auf den mit lösungsgetränkten Longuetten behandelten Inzisionswunden verschwanden die Erreger meist schon nach wenigen Tagen. Dabei kann der erfindungsgemäßen Lösung auch eine Tiefenwirkung zugeschrieben werden.

Die erfindungsgemäße Lösung stellt damit ein wirksames Antiseptikum im Sinne eines bakteriziden und gewebefreundlichen Adjuvans zur Wundbehandlung vor allem bei der chirurgischen Versorgung von akuten und chronischen Knochen- und Weichteilinfektionen dar, wobei gute Behandlungserfolge in vielen Fällen auch ohne Verabreichung systemischer Antibiotika erzielbar waren.

Wegen der zuverlässigen Bakterizide, der guten lokalen und symptomlosen allgemeinen Verträglichkeit der erfindungsgemäßen Lösung steht auch der Anwendung zur prophylaktischen Wundspülung nichts im Wege. Das betrifft auch die intraabdominale Spülung sowie die Spülung anderer Körperhöhlen.und auch die Spülung von Körperhöhlen.

Von der erfindungsgemäßen Lösung ist aber eine bestmögliche und erfolgreiche Wirkung nur dann zu erreichen, wenn die anerkannten chirurgischen Behandlungsgrundsätze (Vaskularität, Stabilität) korrekt eingehalten werden.

**Patentansprüche**

1. Lactatfreie reine Ringerlösung, die zusätzlich 0,1 % eines Konzentrates gelöst enthält, das aus einer 20%-igen wäßrigen Polyhexamethylenbiguanidin-Hydrochlorid-Lösung besteht, in der pro 100 ml 1 g Polyethylenglykol mit einem Molekulargewicht von etwa 4.000 gelöst ist.

2. Ringerlösung nach Anspruch 1 zur lokalen bakteriellen Dekontamination von Wunden auf einen keimfreien oder saprophytischen Zustand.

3. Ringerlösung nach den Ansprüchen 1 und 2 zur Aufrechterhaltung des keimfreien oder saprophytischen Zustandes während der gesamten Heilungsdauer.

4. Ringerlösung nach Anspruch 2 oder 3 mit einem bis auf maximal 0,2 % erhöhten Konzentrationsgehalt zur Dekontamination von Gram-negativ-Erregern (z.B. Pseudomonas) und/oder Anaerobier.

5. Ringerlösung nach einem oder mehreren der vorstehenden Ansprüche als bakterizid wirkendes Medikament zur Anwendung bei therapeutischen Verfahren für die lokale Behandlung von infizierten Wunden.

6. Ringerlösung nach einem oder mehreren der vorstehenden Ansprüche 1 bis 4 als bakterizid wirkendes Medikament für die lokale prophylaktische Behandlung von infektionsgefährdeten Wunden.

7. Ringerlösung nach Anspruch 6 für die prophylaktische Behandlung von infektionsgefährdeten Wunden während der Operation.

8. Ringerlösung nach einem oder mehreren der vorstehenden Ansprüche 1 bis 4 als bakterizid wirkendes Medikament zur Sicherung der lokalen bakteriellen Dekontamination von Wunden während des Heilungsprozesses.

9. Ringerlösung nach einem oder mehreren der vorstehenden Ansprüche zum Tränken von Wundabdekkungsmaterialien, wie Gazen, Tamponaden, Longuetten und Kunststoffmaterialien etc.

Europäisches Patentamt

# EUROPÄISCHER RECHERCHENBERICHT

Nummer der Anmeldung

**EP 90 10 6312**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-3 537 627 (MERCK PATENT GmbH)<br>* Insgesamt *<br>- - - | 1-9 | A 01 N 47/44<br>A 61 K 33/14 //<br>(A 01 N 47/44<br>A 01 N 59:08<br>A 01 N 31:02 ) |
| X | GB-A-1 167 249 (IMPERIAL CHEMICAL INDUSTRIES LTD)<br>- - - - - | | |

| RECHERCHIERTE SACHGEBIETE (Int. Cl.5) |
|---|
| A 01 N<br>A 61 K |

Der vorliegende Recherchenbericht wurde für alle Patentansprüche erstellt

| Recherchenort | Abschlußdatum der Recherche | Prüfer |
|---|---|---|
| Den Haag | 06 November 90 | DONOVAN T.M. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument